# EUROPÄISCHE PATENTSCHRIFT

(11) **EP 0 418 342 B1**
(45) Veröffentlichungstag und Bekanntmachung des Hinweises auf die Patenterteilung: **01.12.1993**
(21) Anmeldenummer: 90904224.4
(22) Anmeldetag: 03.03.1990
(51) Int. Cl.: G01N 33/34, G01N 27/22, A24C 5/58

(54) **EINRICHTUNG ZUR ERFASSUNG AUSREICHENDER BELEIMUNG EINES PAPIERSTREIFENS**
DEVICE FOR DETECTING THE SUFFICIENCY OF AN ADHESIVE COATING ON A STRIPE OF PAPER
DISPOSITIF DE DETECTION DE L'ADHERENCE DE LA COUCHE D'ADHESIF SUR UNE BANDE DE PAPIER

(30) Priorität: 25.03.1989 DE 3909990
(43) Veröffentlichungstag der Anmeldung: 27.03.1991
(73) Patentinhaber: B.A.T. CIGARETTENFABRIKEN GMBH, D-22761 Hamburg (DE)
(72) Erfinder: MASURAT, Heinz, D-2081 Borstel-Hohenraden (DE); GAISSER, Horst, D-2087 Hassloh (DE); MEYER, Meinhard, D-2081 Appen-Unterglinde (DE); PAUTKE, Ingo, D-2000 Hamburg 73 (DE)
(74) Vertreter: Meyer, Ludgerus A., Dipl.-Ing.
(86) Internationale Anmeldenummer: DE9000152
(87) Internationale Veröffentlichungsnummer: WO9011518

(56) Entgegenhaltungen:
- DE-A- 3 143 526
- US-A- 3 043 993
- US-A- 3 713 966

## Beschreibung

Die Erfindung betrifft eine Einrichtung zur Erfassung ausreichender Beleimung eines zu verklebenden Papierstreifens in einer Cigarettenmaschine nach dem Oberbegriff des Anspruchs 1.

Zur Verbindung von Cigarettenfiltern mit dem den Tabak enthaltenden Cigarettenabschnitt ist in der Regel ein Filterbelagpapier vorgesehen, das einerseits den Filter umhüllt und andererseits mit einer überstehenden Kante den den Tabak enthaltenden Cigarettenabschnitt mit dem Filter verbindet. Das Zusammenfügen des Filters mit dem Cigarettenabschnitt erfolgt in der Regel durch Verleimen.

In heute gebäuchlichen Cigarettenmaschinen werden die Cigaretten in der Regel zunächst als Doppel-Cigaretten hergestellt, die dann aufgeschnitten und auseinandergezogen werden. Dann werden in den Zwischenraum zwischen den beiden Cigaretten Doppel-Filter eingereiht. Filter und die beiden seitlichen Cigarettenabschnitte werden dann mit dem zuvor beleimten Filterbelagpapier umhüllt, wobei jeweils ein Seitenbereich des Filterbelagpapiers Filter und Cigarette miteinander verbindet.

Aus bestimmten Gründen sind heute verwendete Filterbelagpapiere häufig mit Zonen erhöhter Luftdurchlässigkeit versehen, um eine Ventilation des Filterbereiches zu ermöglichen. In diesem Bereich darf daher keine Leimschicht auf das Filterbelagpapier aufgetragen sein.

Es hat sich gezeigt, daß es insbesondere bei hohen Produktionsgeschwindigkeiten vorkommen kann, daß die auf das Filterbelagpapier aufgetragene Beleimung bereits vor der Verbindung mit dem Filter bzw. der Cigarette soweit abgetrocknet ist, daß keine ausreichende Verbindung gewährleistet ist. Es kann ferner der Fall eintreten, daß die Beleimungsschicht zu dünn aufgetragen wurde und/oder deren Feuchtegehalt zu gering war. In allen Fällen werden fehlerhafte Cigaretten erzeugt, die im weiteren Produktionsprozeß nicht mehr ohne weiteres auffindbar sind.

Aus der EP 0 300 734 A2 sind ein Verfahren und eine Einrichtung zur Feststellung des Vorhandenseins einer Leimschicht auf einem laufenden Band bekannt. Hier wird die erhöhte Lichtreflexion einer Leimschicht im Vergleich zur Reflexion des unbeleimten Papiers ausgewertet und damit das Vorhandensein einer Beleimung festgestellt.

Dieses Verfahren kann nur das Vorhandensein, jedoch nicht die Stärke einer Leimschicht feststellen. Ferner können Fehlmessungen infolge Verschmutzung der optischen Komponenten auftreten.

Aus der DE-OS 31 43 526 ist ein Verfahren zum überwachen einer ausreichenden Beleimung auf einem Papierstreifen bekannt, bei dem das Vorhandensein ausreichender Beleimung mit Hilfe eines kapazitiven Sensors erfaßt wird.

Diese Einrichtung ist nicht zur Verwendung an Filterbelagpapieren geeignet, die eine Beleimung in Form eines wiederkehrenden Musters aufweisen, da hierbei eine sehr genaue Führung des Papierstreifens gegenüber dem Sensor erforderlich ist und der Sensor nicht in der Lage ist, ein wiederkehrendes Muster von nicht gewünschten Unterbrechungen der Beleimung zu unterscheiden.

Aus der US-A 3 043 993 ist eine Leimprüfmaschine mit kapazitiven Sensoren auf der der Beleimung einer Papierbahn gegenüberliegenden Seite bekannt. Die Sensoren sind in Laufrichtung des Papiers in einer Gleitplatte hintereinander angeordnet und decken einen großen Teil der Papierstreifenbreite ab. Beleimung auf Papierstreifen mit wiederkehrendem Leimmuster können mit dieser Einrichtung nicht ausgewertet werden.

Die US-A 3 713 966 offenbart eine Vorrichtung zur Feuchtigkeitsmessung von Papier mit Hilfe kapazitiver Sensoren. Die Sensoren sind quer zur Papierlaufrichtung angeordnet, um dem Verhältnis der großen Papierbreite zur Sensorengröße Rechnung zu tragen.

Keine der beiden letztgenannten Erfindungen sieht eine Anordnung nach Art der Erfindung vor, die verhindert, daß der Papierstreifen auf der Gleitplatten flatternd und damit unsicher und beschädigungsgefährdet gleitet.

Der Erfindung liegt die Aufgabe zugrunde, eine Einrichtung zur Erfassung ausreichender Beleimung eines zu verklebenden Papierstreifens anzugeben, mit deren Hilfe sowohl das Vorhandensein als auch der Zustand einer insbesondere in wiederkehrenden Muster aufgetragenen Beleimung kurz vor dem Verkleben des Papierstreifens in einer Cigarettenmaschine korrekt feststellbar ist.

Diese Aufgabe wird durch die im Anspruch 1 angegebenen Erfindung gelöst. Vorteilhafte Weiterbildungen der Erfindung sind in Unteransprüchen angegeben.

Durch Verwendung der Erfindung ist es möglich, unmittelbar vor der Verbindung eines Filterbelagpapiers mit dem Filter bzw. Cigarettenenden festzustellen, ob die zu dieser Verbindung vorgesehene Beleimung des Filterbelagpapiers ausreichend ist oder keine ausreichende Verbindung mehr zuläßt.

Der Feuchtegehalt der Beleimung wird durch kapazitive Sensoren festgestellt. Bei nicht ausreichender Feuchte infolge zu starken Abtrocknens oder zu dünner Leimschicht wird ein Fehlersignal erzeugt, das die auf diese Weise verleimten Cigaretten aus dem weiteren Verarbeitungslauf ausscheidet.

Durch die Anordnung der Sensoren unterhalb des Filterbelagpapiers ist gewährleistet, daß diese immer sauber bleiben, ohne daß, insbesondere bei Stillstand, ein Ankleben des Leims auf den Sensoren möglich wäre. Vorzugsweise sind die Sensoren unmittelbar vor der Schneideeinrichtung des Filterbelagpapiers angeordnet, um den Zustand der Beleimung möglichst nahe am Ort des Verklebens erfassen zu können.

Das Vorhandensein einer Gleitplatte sichert eine exakte Führung des Filterbelagpapiers, und damit insbesondere die Einhaltung eines konstanten Abstandes der Sensoren vom Filterbelagpapier. Ein eventuelles Schwingen oder Flattern des Filterbelagpapiers wird durch Kanäle verhindert, die in der Oberfläche der Gleitplatte rillenförmig angeordnet und in einem Winkel von 10 - 80° gegen die Laufrichtung des Filterbelegpapiers gerichtet sind.

In den Unteransprüchen 2 - 8 sind bevorzugte Weiterbildungen der Erfindung zur sicheren Verhinderung von Schwingen oder Flattern des Filterbelagpapiers angegeben.

Die Unteransprüche 9 und 10 geben besondere Ausführungsformen der Erfindung bei Einsatz an einem Filterbelagpapier an, auf dem die Beleimung in wiederkehrendem Muster aufgetragen ist.

Die Erfindung erlaubt eine genaue Dosierung der Beleimung und eine verbesserte Fehlererkennung, so daß der Ausschuß verringert werden kann und damit die Produktivität erhöht wird.

Die Erfindung wird nachstehend anhand eines Ausführungsbeispiels näher erläutert. Es zeigen
- Fig. 1: eine schematische Seitenansicht eines Teils einer Cigarettenmaschine, in der die Einrichtung nach der Erfindung angeordnet ist,
- Fig. 2: eine Aufsicht auf ein Filterbelagpapier mit räumlich zugeordneten Sensoren,
- Fig. 3: eine Ansicht von Halterungen für Sensoren,
- Fig. 4: eine Aufsicht auf eine Haltung für Sensoren mit zusätzlichen Luftkanälen,
- Fig. 5: eine Seitenansicht einer Gleitplatte
- Fig. 6: ein Blockdiagramm einer Auswerteschaltung.

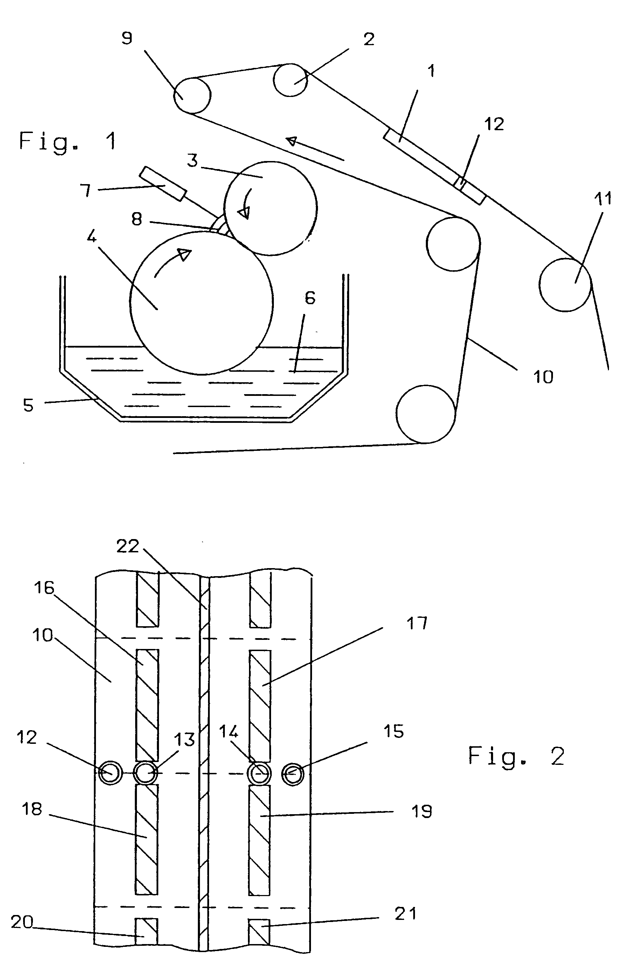

Fig. 1 zeigt die Beleimungseinrichtung an einer Cigarettenmaschine. Es ist ein Leimtopf 5 vorgesehen, der aus einem Vorratsbehälter ständig mit Leim befüllt wird. Aus diesem Topf 5 wird über eine sich drehende Tauchwalze 4 Leim aufgenommen und auf eine Leimwalze 3 übertragen. Von der Leimwalze 3 kann der Leim auf das an die Leimwalze 3 andrückbare Filterbelagpapier 10 überführt werden. Das Filterbelagpapier 10 läuft dabei als endloser Streifen über mehrere Umlenkrollen zu einer schwenkbaren Umlenkrolle 9, mit deren Hilfe das Belagpapier 10 an die Leimwalze 3 andrückbar ist. Nach Übertragung der Beleimung auf das Filterbelagpapier 10 läuft das Filterbelagpapier in der Filteransetzmaschine 10 zur Verbindungsstelle zwischen Filter und Cigarette und umhüllt dort den Filter und verbindet diesen mit der Cigarette.

Der aus dem Leimtopf 5 durch die Tauchwalze 4 aufgenommene Leim 6 bildet beim Übergang auf die Leimwalze 3 einen Leimwulst 8, der über einen Infrarot-Sensor erfaßbar ist. An dieser Stelle kann festgestellt werden, ob überhaupt Leim aus dem Leimtopf aufgenommen wurde.

Die Erfindung sieht vor, daß kurz vor der Stelle, an der die Filter an die Cigaretten angesetzt werden, eine Einrichtung zur Erfassung einer ausreichenden Beleimung des Papierstreifens, in diesem Fall des Filterbelagpapiers vorgenommen wird.

Dazu ist eine ein- oder zweiteilige Gleitplatte 1 vorgesehen, über die das Filterbelagpapier 10 läuft, um ein Flattern des Papiers zu vermeiden. Diese Platte 1 kann eine eigens in die Maschine integrierte Platte sein, es kann zu diesem Zweck auch die bei älteren Maschinen vorhandene Schleifheizung verwendet werden, die früher bei Verwendung schlecht trocknender Leime notwendig war.

Die Gleitplatte weist vorzugsweise eine Wölbung mit einem Radius von > 50 cm, insbesondere 80 cm auf.

In Ausnehmungen der Platte 1 sind quer zur Laufrichtung des Filterbelagpapiers in einer Reihe angeordnete Sensoren 12 eingesetzt. Das Filterbelagpapier läuft damit mit seiner nicht mit der Beleimung versehenen Seite über die Sensoren.

Die Sensoren sind kapazitive Sensoren, die in ihrem Meßbereich auf veränderte Feuchtegehalte in ihrem Meßbereich reagieren. Daher können die Sensoren die Feuchte der Beleimung auf der dem Filterbelagpapier gegenüberliegende Seite erfassen und auswerten.

Fig. 2 zeigt einen Abschnitt eines Filterbelagpapiers in Aufsicht. Von der Beleimung sind die Bereiche 16 bis 21 ausgenommen, die die Ventilationszonen im Filter der fertigen Cigarette bilden. In den übrigen Bereichen, mit Ausnahme des Trennbereiches 22 ist eine vollständige Beleimung erforderlich. Zu dessen Erfassung sind auf der dem Leim gegenüberliegenden Seite des Belagpapiers die Sensoren 12 bis 15 vorgesehen, die jeweils die Beleimung des Filterbelagpapiers in einem schmalen Streifen erfassen. Die Sensoren 12 und 15 erfassen dabei die Außenbereiche des Filterbelagpapiers, die den Bereich darstellen, der für die Verbindung des Filters mit der Cigarette vorgesehen ist. Die Sensoren 13 und 14 erfassen den Bereich des Filterbelagpapiers, in dem die Ventilationszonen vorgesehen sind. Jeweils zwischen zwei Ventilationsabschnitten ist eine Beleimung vorgesehen, die bei der Umwicklung des Filterbelagpapiers um den Filter die Kantenverbindung bewirkt. Für eine Cigarette ist jeweils ein Abschnitt des Filterbelagpapiers vorgesehen, der zwischen zwei quergestrichelten Linien liegt.

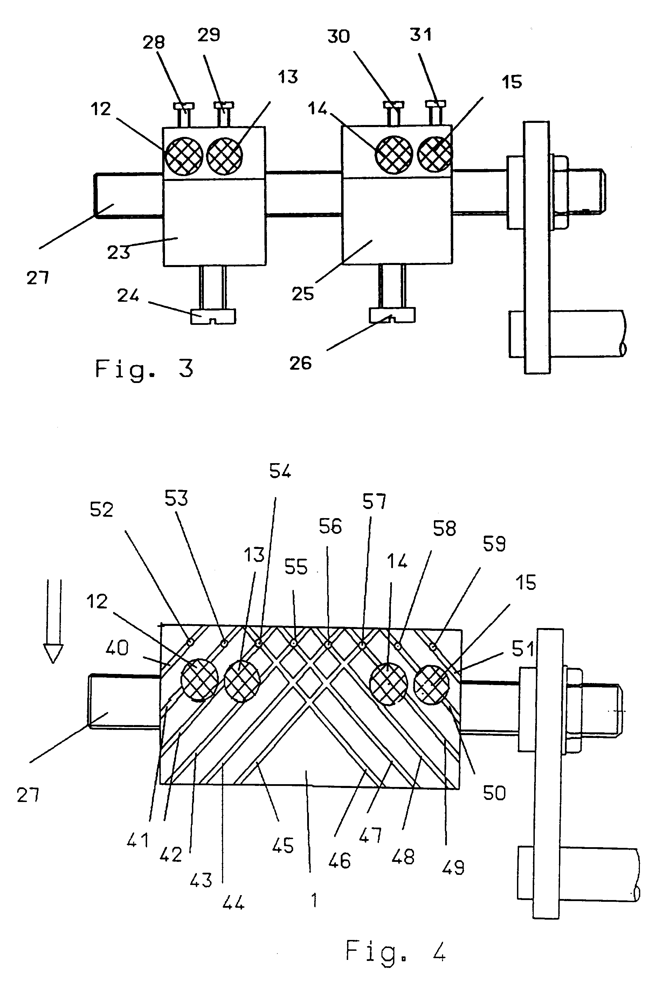

Fig. 3 zeigt die Halterung der Sensoren. Es sind zwei die Gleitplatte 1 haltende Halter 23 und 25 vorgesehen, die über Stellschrauben 24 und 26 in Axialrichtung einer Welle 27 verschiebbar sind. Die Sensoren 12 bis 15 selbst sind in Axialrichtung in Ausnehmungen der Halter 23 und 25 verschieblich angeordnet und über Stellschrauben 28 bis 31 festlegbar. Die Sensoren können auf diese Weise exakt auf die Breite und die Bespurung des Filterbelagpapiers eingestellt werden. In einem besonderen Ausführungsbeispiel der Erfindung sind die Sensoren etwas unterhalb der Oberfläche der Halter angeordnet. In den danach gebildeten Raum zwischen Sensoren und Oberfläche ist dielektrisches Material eingesetzt, um eine Verschmutzung der Räume zu verhindern. Durch diese Anordnung kann die Meßsicherheit bei einem eventuellen Flattern des Filterbelagpapiers erhöht werden.

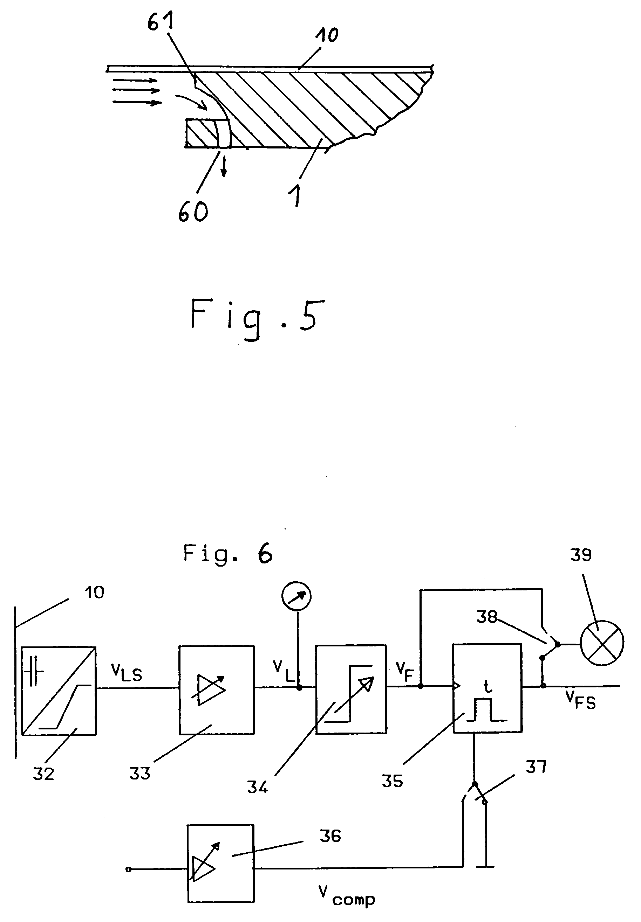

Fig. 4 zeigt eine einteilige Gleitplatte in Aufsicht. In einem Winkel von etwa 45° zur Laufrichtung des Filterbelagpapiers sind mehrere einzelne Kanäle (40 - 51) mit einer Breite von beispielsweise 1 mm und einer Tiefe von 2 mm in die Oberfläche der Gleitplatte 1 eingefräst. Die Kanäle führen bis an die Kanten der Gleitplatte, um einen freien Ein- und Austritt der Luft zu ermöglichen. Die Kanäle sorgen dafür, daß sich zwischen Gleitplatte und Filterbelagpapier kein Luftpolster bildet, das ein Flattern des Filterbelagpapiers verursachen und damit eine konkrete Messung verhindern könnte.

Zusätzlich oder alternativ kann vorgesehen sein, an die Kanäle eine Saugquelle 52 - 59 anzuschließen, um eine gewisse Andruckkraft des Filterbelagpapiers an die Gleitplatte zu erzeugen.

Es kann auch vorgesehen sein, anstelle einer festen Gleitplatte ein Lochrasterblech vorzusehen, durch das die Luft unmittelbar entweichen kann.

Fig. 5 zeigt eine Gleitplatte in Seitenansicht. Zur Verhinderung eines Luftzwischenraums zwischen Gleitplatte und Filterbelagpapier ist hier eine scharfkantige Auflaufkante 61 vorgesehen, an der am Filterbelagpapier 10 anhaftende Luft abgeleitet wird. Dies kann durch Absaugen über den Absaugkanal 60 noch unterstüzt werden.

Fig. 6 zeigt ein Blockdiagramm der bei der Erfindung verwendeten Auswerteschaltung. Ein kapazitiver Sensor 12 erfaßt den Zustand der Beleimung auf dem vorbeilaufenden Filterbelagpapier, indem sich in Abhängigkeit vom Feuchtegehalt der Beleimung das Dielektrikum im Meßbereich des Sensors ändert.

Die erfaßte Kapazität wird über den Wandler 32 in eine Spannung Vₗₛ umgewandelt, die über den Verstärker 33 als Vₗ Signal verstärkt wird. Im Anschluß an den Verstärker 33 kann das gemessene Signal zur Anzeige gebracht werden, wodurch sich die "Leimkraft" meßtechnisch erfassen läßt. In der Komparatorstufe 34 wird ein Gleichspannungsanteil abgetrennt. Die von dem Sensor erfaßten Signale werden dann in Rechtecksignale V_{f} umgewandelt. Die V_{f}-Signale werden auf eine Impulsstufe übertragen, die von der ansteigenden Flanke des V_{f}-Signals getriggert wird. Der erzeugte Impuls V_{fs} weist eine Länge mit der Zeitdauer t auf, die der Laufgeschwindigkeit der Filteransetzmaschine angepaßt ist, z. B. 7.000 Cigaretten pro Minute.

Für die Sensoren, die sich in den Bereichen des Filterbelagpapiers befinden, die eine unterbrechungsfreie Bespurung aufweisen, gilt folgendes: Sobald die Beleimung nicht mehr ausreicht oder eine Lücke in der Beleimung auftritt, wird im Komparator 34 ein Spannungssprung erzeugt, der in der nachfolgenden Impulsstufe 35 ein Triggersignal V_{fs} erzeugt, das ein Fehlersignal darstellt. Dieses ist über eine Anzeige 39 darstellbar oder kann als Steuersignal an die Filteransetzmaschine gegeben werden, um für eine bestimmte Zeitdauer die hergestellten Cigaretten aus dem Produktionslauf auszustoßen oder die Maschine insgesamt abzuschalten. Die Anzeige 39 kann auch über den Schalter 38 unmittelbar an das aus dem Komparator abgegebene Signal V_{f} angeschaltet werden.

Für den Bereich des Filterbelagpapiers, der Ventilationszonen aufweist, wird durch die Sensoren eine Pulsfolge erzeugt, aus der nicht ohne weiteres ein Fehlersignal ableitbar ist. Daher ist vorgesehen, daß das in der Impulsstufe 35 erzeugte Rechtecksignal eine derartige Länge aufweist, daß bei fehlerloser Beleimung jeweils vor Ende des erzeugten Impulses ein neuer Triggerimpuls auftritt, der die Impulsstufe nachtriggert, so daß der erzeugte Impuls ständig verlängert wird. Erst bei Ausfall eines Triggerimpulses aufgrund fehlerhafter Beleimung endet der in der Impulsstufe erzeugte Impuls, so daß der Abfall des Impulses als Fehlersignal zur Ansteuerung der Filteransetzmaschine ausgewertet werden kann.

Das Signal V_{f} kann auch verwendet werden, um die Beleimung mit dem Ort des Schneidens des Belagpapiers für die einzelnen Cigaretten zu synchronisieren. Damit läßt sich eine exakt definierte Schnittstelle auf dem Belagpapier festlegen.

Die Zeitdauer des in der Impulsstufe 35 erzeugten Impulses kann über einen Schalter 37 konstant eingestellt werden. Vorzugsweise wird die Länge des Impulses jedoch von der Drehzahl der Filteransetzmaschine abhängig gemacht, indem ein Steuersignal über einen Verstärker 36 auf die Impulsstufe 35 zur Bestimmung der Pulslänge gegeben wird. Die Schaltung arbeitet daher auch bei unterschiedlichen Drehzahlen, insbesondere auch im Anfahrbereich der Maschine einwandfrei.

| Bezugszeichenliste | | | |
|---|---|---|---|
| 1 | Platte | 60 | Absaugkanal |
| 2 | Rolle | 61 | Auflaufkante |
| 3 | Leimwalze | | |
| 4 | Tauchwalze | | |
| 5 | Topf | | |
| 6 | Leim | | |
| 7 | Sensor | | |
| 8 | Leimwulst | | |
| 9 | Umlenkrolle | | |
| 10 | Papierstreifen | | |
| 11 | Umlenkrolle | | |
| 12 - 15 | Sensoren | | |
| 16 - 21 | Ventilationsbereich | | |
| 22 | Trennspur | | |
| 23 | Halter | | |
| 24 | Stellschraube | | |
| 25 | Halter | | |
| 26 | Stellschraube | | |
| 27 | Welle | | |
| 28 - 31 | Stellschraube | | |
| 32 | Wandler | | |
| 33 | Verstärker | | |
| 34 | Komparator | | |
| 35 | Impulsstufe | | |
| 36 | Verstärker | | |
| 37 | Schalter | | |
| 38 | " | | |
| 39 | Lampe | | |
| 40 - 51 | Kanäle | | |
| 52 - 59 | Absauganschlüsse | | |

## Patentansprüche

1. Einrichtung zur Erfassung ausreichender Beleimung eines zu verklebenden Papierstreifens (10) in einer Cigarettenmaschine mit wenigstens einem das Vorhandensein einer Beleimung erfassenden kapazitiven Sensor (12) und einer Steuereinrichtung zur Auswertung des Sensorsignals (Vₗₛ), wobei der Sensor (12 - 15) auf der der Beleimung gegenüberliegenden Seite zur Erfassung der Feuchte der Beleimung gegen den laufenden Papierstreifen (10) gerichtet ist, und bei nicht ausreichendem Feuchtegehalt ein Fehlersignal (V_{fs}) zur Steuerung der Cigarettenmaschine abgegeben wird, wobei mehrere Sensoren (12 - 15) in einer Reihe quer zum laufenden Filterbelagpapier angeordnet sind und die Sensoren in Ausnehmungen einer Gleitplatte (1) aufgenommen sind, über die das Filterbelagpapier läuft, dadurch gekennzeichnet, daß die Gleitplatte an der Oberfläche rillenförmige Kanäle (40 - 51) aufweist, die in einem Winkel von 10°-80° gegen die Laufrichtung des Filterbelagpapiers (10) gerichtet sind.

2. Einrichtung nach Anspruch 1, dadurch gekennzeichnet, daß die Gleitplatte in Laufrichtung des Filterbelagpapiers eine Wölbung mit einem Radius >50 cm aufweist.

3. Einrichtung nach Anspruch 1, dadurch gekennzeichnet, daß die Kanäle (40 - 51) in einem Winkel von 45° gegen die Laufrichtung des Filterbelagpapiers (10) gerichtet sind.

4. Einrichtung nach Anspruch 1, dadurch gekennzeichnet, daß die Gleitplatte (1) über die Bandbreite des Filterbelagpapiers mehrere Absauganschlüsse (52 - 59) aufweist.

5. Einrichtung nach Anspruch 4, dadurch gekennzeichnet, daß die Absauganschlüsse (52 - 59) in den Kanälen (40 - 51) münden.

6. Einrichtung nach Anspruch 1, dadurch gekennzeichnet, daß die Gleitplatte (1) eine Lochrasterplatte ist.

7. Einrichtung nach einem oder mehreren der vorhergehenden Ansprüche, dadurch gekennzeichnet, daß die Gleitplatte (1) eine scharfkantige Auflaufkante (61) aufweist.

8. Einrichtung nach Anspruch 1, dadurch gekennzeichnet, daß die Sensoren im Abstand unter der Oberfläche der Gleitplatte angeordnet sind und daß zwischen den Sensoren und der Oberfläche der Gleitplatte ein dielektrisches Material eingebracht ist.

9. Einrichtung nach einem oder mehreren der vorhergehenden Ansprüche, unter Verwendung eines Filterbelagpapiers, auf dem die Beleimung in wiederkehrendem Muster (16 - 21) auf dem Papierstreifen (10) aufgetragen ist, dadurch gekennzeichnet, daß das Sensorsignal auf eine Schaltung zur Erzeugung eines nachtriggerbaren Impulses mit vorbestimmter Impulsdauer geführt ist, und daß bei Abfall des Impulses aufgrund nicht ausreichenden Feuchtigkeitsgehalts am Ende eines Impulses das Fehlersignal erzeugt wird.

10. Einrichtung nach Anspruch 9, dadurch gekennzeichnet, daß die Impulsdauer von der Maschinengeschwindigkeit abhängig ist.

## Claims

1. A device in a cigarette machine for detecting adequate application of glue to a paper strip (10) to be glued, having at least one capacitive sensor (12) detecting the presence of an application of glue and a control device for evaluating the sensor signal (Vₗₛ), wherein the sensor (12 - 15) at the side opposite the application of glue is directed towards the running paper strip (10) for the purpose of detecting the moistness of the application of glue, and a fault signal (V_{fs}) for controlling the cigarette machine is generated in the event of an inadequate moisture content, wherein a plurality of sensors (12 - 15) is disposed is a row transversely to the running filter covering paper and the sensors are accommodated in recesses in a slide plate (8) across which the filter covering paper runs, characterised in that the surface of the slide plate has grooved-shaped channels (40 - 51) which are directed at an angle of from 10° - 80° in the opposite direction to the running direction of the filter covering paper (10).

2. A device as claimed in claim 1, characterised in that the slide plate has in the running direction of the filter covering paper a curvature having a radius greater than 50 cm.

3. A device as claimed in claim 1, characterised in that the channels (40 -51) are directed at an angle of 45° in the opposite direction to the running direction of the filter covering paper (10).

4. A device as claimed in claim 1, characterised in that the slide plate (1) has a plurality of suction connections (52 - 59) across the width of the strip of filter covering paper.

5. A device as claimed in claim 4, characterised in that the suction connections (52 - 59) open into the channels (40 - 51).

6. A device as claimed in claim 1, characterised in that the slide plate (1) is a perforated raster plate.

7. A device as claimed in one or several of the preceding claims, characterised in that that the slide plate (1) has a sharp-edged run-on edge (61).

8. A device as claimed in claim 1, characterised in that the sensors are disposed at a distance below the surface of the slide plate, and that a dialectric material is placed between the sensors and the surface of the slide plate.

9. A device as claimed in one or several of the preceding claims, using a filter covering paper on which the glue is applied to the paper strip (10) in a recurrent pattern (16-21), characterised in that the sensor signal is applied to a circuit for generating a subsequently triggerable pulse having a predetermined duration, and that, in the event of decay of the pulse due to an inadequate moisture content, the fault signal is generated at the end of a pulse.

10. A device as claimed in claim 9, characterised in that the pulse duration is dependent upon the speed of the machine.

## Revendications

1. Dispositif pour détecter un encollage suffisant d'une bande de papier (10) à coller, dans une machine à cigarettes, comportant au moins un détecteur capacitif (12) détectant la présence d'un encollage, et un dispositif de commande pour l'analyse du signal de détecteur (Vₗₛ), étant précisé que le détecteur (12-15) prévu sur le côté opposé à l'encollage est dirigé vers la bande de papier (10) qui défile, en vue de détecter l'humidité de l'encollage, qu'en présence d'un degré d'humidité insuffisant, un signal d'erreur (V_{fs}) est envoyé à la commande de la machine à cigarettes, que plusieurs détecteurs (12-15) sont disposés en ligne, transversalement par rapport au papier de garniture filtrant qui défile, et qu'ils sont logés dans des cavités d'une plaque de glissement (8) sur laquelle le papier de garniture filtrant passe, caractérisé en ce que la plaque de glissement présente, sur sa surface, des canaux en forme de rainures (40-51) qui sont orientés suivant un angle de 10°-80° par rapport au sens de défilement du papier de garniture filtrant (10).

2. Dispositif selon la revendication 1, caractérisé en ce que la plaque de glissement présente, dans le sens de défilement du papier de garniture filtrant, une courbure suivant un rayon > 50 cm.

3. Dispositif selon la revendication 1, caractérisé en ce que les canaux (40-51) sont orientés suivant un angle de 45° par rapport au sens de défilement du papier de garniture filtrant (10).

4. Dispositif selon la revendication 1, caractérisé en ce que la plaque de glissement (1) comporte plusieurs raccords d'aspiration (52-59) sur la largeur de bande du papier de garniture filtrant.

5. Dispositif selon la revendication 4, caractérisé en ce que les raccords d'aspiration (52-59) débouchent dans les canaux (40-51).

6. Dispositif selon la revendication 1, caractérisé en ce que la plaque de glissement (1) est une plaque quadrillée perforée.

7. Dispositif selon l'une au moins des revendications précédentes, caractérisé en ce que la plaque de glissement (1) présente un bord d'attaque à arête vive (61).

8. Dispositif selon la revendication 1, caractérisé en ce que les détecteurs sont disposés à une certaine distance sous la surface de la plaque de glissement, et en ce qu'un matériau diélectrique est inséré entre les détecteurs et la surface de la plaque de glissement.

9. Dispositif selon l'une au moins des revendications précédentes, utilisant un papier de garniture filtrant sur lequel l'encollage est appliqué suivant un motif répété (16-21) sur la bande de papier (10), caractérisé en ce que le signal de détecteur est transmis à un circuit destiné à générer une impulsion apte à être re-déclenchée présentant une durée d'impulsion prédéfinie, et en ce que, lors d'une diminution d'impulsion en raison d'un degré d'humidité insuffisant, à la fin d'une impulsion, le signal d'erreur est généré.

10. Dispositif selon la revendication 9, caractérisé en ce que la durée d'impulsion dépend de la vitesse de la machine.
